(19) Europäisches Patentamt
European Patent Office
Office européen des brevets

(11) Numéro de publication: **0 036 378**
**B1**

# (12) FASCICULE DE BREVET EUROPEEN

(45) Date de publication du fascicule du brevet: **06.07.83**

(51) Int. Cl.³: **C 07 C 37/07**, C 07 C 39/27

(21) Numéro de dépôt: **81420032.5**

(22) Date de dépôt: **06.03.81**

## (54) Procédé de préparation de chlorophénols.

(30) Priorité: **17.03.80 FR 8005876**

(43) Date de publication de la demande: **23.09.81 Bulletin 81/38**

(45) Mention de la délivrance du brevet: **06.07.83 Bulletin 83/27**

(84) Etats contractants désignés: **AT BE CH DE GB IT LI LU NL SE**

(56) Documents cités:
**DE-C-64 426**
**FR-A-2 002 799**
**FR-A-2 079 575**
**FR-A-2 125 280**
**NL-A-7 509 917**
**US-A-3 532 740**
**US-A-3 660 505**
**JOURNAL OF THE AMERICAN CHEMICAL SOCIETY, vol. 75, 5 juin 1953, WASHINGTON D. C. (US) F. MARSHALL BERINGER et al.: »The aromatization and rearrangement of cyclic ketones. III 2,3,5- and 3,4,5-Trimethylphenol from Isophorone« pages 2633—2635**
**CHEMICAL ABSTRACTS, vol. 89, no. 15, 9 octobre 1978, ref. 129172z, page 559 COLUMBUS, OHIO (US)**

(73) Titulaire. **RHONE-POULENC SPECIALITES CHIMIQUES, "Les Miroirs" 18, Avenue d'Alsace, F-92400 Courbevoie (FR)**

(72) Inventeur: **Bourdon, Jacques, 1, Rue Jacques Level, F-04600 Saint-Auban (FR)**

(74) Mandataire: **Rioufrays, Roger et al, RHONE-POULENC RECHERCHES Centre de Recherches de Saint-Fons Service Brevets B.P. 62, F-69190 Saint-Fons (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

Procédé de préparation de chlorophenols

La présente invention a pour objet un procédé pour la préparation de chlorophénols; elle concerne plus particulièrement la préparation de chlorophénols à partir de cyclohexanones chlorées.

On connait dans l'art antérieur des procédés de ce type.

Le plus ancien consiste pour la préparation du dichloro-2,6 phénol à porter à une température de 260–270°C la tétrachloro-2,2,6,6 cyclohexanone (Acta Chemica Scandinavia 1950 4 200-4). Ce procédé conduit cependant à des rendements très faibles.

Un article du Journal of American Chemical Society 75 (1953) pages 2633 à 2635 indique que des phénols halogénés ont été obtenus à partir de cyclohexènones halogénées par chauffage; mais on note une migration des substituants du cycle cyclohexène.

Le brevet français n° 70/04082 (publié sous le n° 2 079 575) décrit un procédé de déshydrochloration de divers composés en présence de phosphate ou de phosphonate d'ammonium quaternaire. A partir de la dibromo-2,6 cyclohexanone on obtient le phénol.

Le brevet américain décrit le réarrangement de paraalkénylquinones substituées en paraalkénylphénols substitués correspondants en présence de trialkylphosphines ou d'alkylamines tertiaires.

Un article des IZV. AKAD. NAUK SSSR, Ser. Khim. 1978 (7) 1660–1661 décrit la réduction par la monophénylphosphine de quinones, substituées notamment par des atomes de chlore ou de brome, en hydroquinones substituées correspondantes.

Un autre porcédé est décrit dans la demande de brevet français 7 201 083 publiée sous le numéro 2 125 280. Il consiste à porter la tétrachloro-2,2,6,6 cyclohexanone ou la trichloro-2,2,6 cyclohexanone à une température comprise entre 100 et 250° C en présence d'une amine, d'un amide, d'une urée ou d'un de leurs sels d'acides, pour obtenir respectivement le dichloro-2,6 phénol et l'orthochlorophénol. Il apparait à la lecture des exemples de cette demande de brevet et d'après les propres constatations de la demanderesse, que les meilleures rendements sont obtenus à 200° C.

Bien que ce dernier procédé permette par rapport au premier d'une part d'améliorer le rendement, et d'autre part d'abaisser sensiblement la température réactionnelle, il est clair pour l'homme de l'art qu'il serait très intéressant de pouvoir disposer d'un procédé qui, tout en conservant les mêmes rendements, pourrait être mis en oeuvre à une température plus faible avec une vitesse plus grande.

Les travaux de la demanderesse ont conduit à un tel procédé.

La présente invention a donc pour objet un procédé de préparation de chlorophénols caractérisé en ce qu'on déshydrochlore thermiquement à une température supérieure à environ 120°C une chlorocyclohexanone de formule générale:

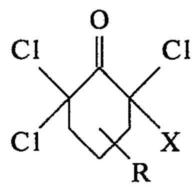

(I)

dans laquelle X représente un atome d'hydrogène ou un atome de chlore, et R représente au moins un radical choisi parmi le groupe comprenant H, les radicaux alkyle ayant de 1 à 5 atomes de carbone en présence d'un catalyseur de formule générale:

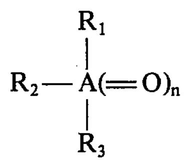

(II)

dans laquelle:

- A représente le phosphore ou l'arsenic;
- $R_1$ et $R_2$ identiques ou différents représentent un atome d'hydrogène ou un radical choisi parmi le groupe comprenant les radicaux alkyle contenant de 1 à 12 atomes de carbone, les radicaux phényle et les radicaux:

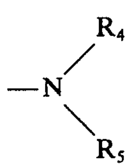

  où $R_4$ et $R_5$ identiques ou différents représentent H ou un radical alkyle ayant de 1 à 5 atomes de carbone environ;
- $R_3$ représente un radical choisi parmi le groupe comprenant les radicaux alkyle contenant de 1 à 12 atomes de carbone, les radicaux phényle et les radicaux:

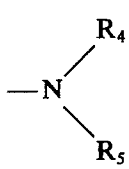

  où $R_4$ et $R_5$ ont la même signification que ci-dessus;
- n est égal à 0 ou 1.

Bien évidemment, le catalyseur retenu doit

avoir une température d'ébullition supérieure à la température à laquelle s'effectue la réaction.

Selon un mode de réalisation préférentiel de l'invention, $R_1$ et $R_2$ identiques ou différents représentent un atome d'hydrogène ou un radical choisi parmi le groupe comprenant les radicaux alkyle ayant de 1 à 4 atomes de carbone et le radical phényle, $R_3$ représentant un radical choisi parmi le groupe comprenant les radicaux alkyle ayant de 1 à 4 atomes de carbone et le radical phényle.

On peut citer comme exemples de phosphines et d'oxyde de phosphines pouvant être mis en oeuvre dans le cadre de la présente invention, les composés suivants: tributylphosphine, triphénylphosphine, diphénylméthylphosphine, diphénylbutylphosphine, diphényléthylphosphine, oxyde de triphénylphosphine, oxyde de triéthylphosphine, oxyde de tributylphosphine, hexaméthylphosphorotriamide.

Selon un mode de réalisation du procédé de l'invention, particulièrement préféré, on utilise la triphénylphosphine.

On peut citer comme exemples d'arsines et d'oxydes d'arsines pouvant être utilisés dans le cadre de la présente invention, les composés suivants: diphénylarsine, triphénylarsine, tributylarsine, oxyde de triphénylarsine, oxyde de tributylarsine.

Selon un autre mode de réalisation du procédé selon l'invention, particulièrement préféré, on utilise la triphénylarsine.

La réaction est de préférence effectuée à une température comprise entre environ 140° C et environ 180° C. Plus particulièrement, on opère à environ 160° C.

On utilise de préférence entre environ 0,1 et environ 10% en poids de catalyseur par rapport à la chlorocyclohexanone de départ. Encore plus préférentiellement, on en utilise entre environ 0,5 et environ 2%.

Bien que l'on préfère opérer sous pression atmosphérique, des pressions supérieures ou inférieures à la pression atmosphérique ne sont pas exclues du domaine de l'invention.

La réaction est généralement effectuée sans solvant mais l'utilisation d'un solvant n'est pas exclue.

On peut citer comme exemples de produits de départ de formule I les composés suivants: la tétrachloro-2,2,6,6 cyclohexanone, la trichloro-2,6,6 cyclohexanone, la méthyl-4 tétrachloro-2,2,6,6 cyclohexanone, la méthyl-3 tétrachloro-2,2,6,6 cyclohexanone.

Les produits obtenus selon le procédé de l'invention ont pour formule générale:

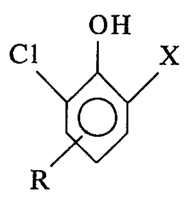

(III)

où X et R ont la signification précédente.

On peut citer comme exemple de formule III les composés suivants: le dichloro-2,6 phénol, l'orthochlorophénol, le méthyl-4 dichloro-2,6 phénol, le méthyl-3 dichloro-2,6 phénol.

Les chlorocyclohexanones de formule I sont obtenues de façon classique par l'homme de l'art, par exemple en chlorant la cyclohexanone correspondante.

D'autres caractéristiques et avantages de la présente invention apparaitront plus clairement à la lecture des exemples qui vont suivre que ne sauraient en aucune manière être considérés comme limitant de façon quelconque l'invention.

## Exemple 1

Dans un réacteur équipé d'une agitation et d'une réfrigérant on charge 236 g (1 mole) de tétrachloro-2,2,6,6 cyclohexanone et 2,5 g (0,01 mole) de triphénylphosphine. On chauffe à 150 – 180° C sous agitation; on arrête la réaction lorsque le dégagement d'acide chlorhydrique cesse. La masse réactionnelle est alors distillée sous pression réduite ($Eb_{20} = 100°$ C), on obtient 137,5 g de dichloro-2,6 phénol.

- Rendement en dichloro-2,6 phénol brut = 89,3%
- Rendement en dichloro-2,6 phénol distillé = 84,3%

## Exemple 2

Dans le réacteur utilisé à l'exemple 1, on charge 190 g (0,8 mole) de tétrachloro-2,2,6,6 cyclohexanone, et 3 g (0,065 mole) de triphényl arsine. On chauffe à 150 – 180° C sous agitation. On traite la masse réactionnelle comme dans l'exemple 1. On obtient 103,4 g de dichloro-2,6 phénol.

- Rendement en dichloro-2,6 phénol brut = 83,3%
- Rendement en dichloro-2,6 phénol distillé = 79,3%

## Exemple 3

Dans le réacteur utilisé à l'exemple 1, on charge 118 g (0,5 mole) de tétrachloro-2,2,6,6 cyclohexanone, et 1 g (0,006 mole) d'hexaméthylphosphorotriamide. On chauffe à 150 – 180° C sous agitation; on arrête la réaction lorsque le dégagement d'acide chlorhydrique cesse. La masse réactionnelle est alors distillée sous pression réduite ($Eb_{20} = 100°$ C), on obtient 52 g de dichloro-2,6 phénol.

- Rendement en dichloro-2,6 phénol = 63,8%

Exemple 4

Dans le réacteur utilisé à l'exemple 1, on charge 222 g (1,1 mole) de trichloro-2,2,6 cyclohexanone, et 2,5 g (0,01 mole) de triphénylphosphine. On chauffe à 175° C sous agitation; on arrête la réaction lorsque le dégagement d'acide chlorhydrique cesse. La masse réactionnelle est alors distillée sous pression réduite ($Eb_{10} = 55°$ C) on obtient 106 g de chloro-2 phénol.

— Rendement en chloro-2 phénol = 75%

Exemple 5

Dans le réacteur utilisé à l'exemple 1, on charge 120 g (0,48 mole) de tétrachloro-2,2,6,6 méthyl-4 cyclohexanone et 2 g (0,008 mole) de triphénylphosphine. On chauffe à 160 – 170° C sous agitation; on arrête la réaction lorsque le dégagement d'acide chlorhydrique cesse. La masse réactionnelle est distillée sous pression réduite, on obtient 49 g de dichloro-2,6 méthyl-4 phénol.

— Rendement en dichloro-2,6 méthyl-4 phénol = 57,7%.

**Revendications**

1. Procédé de préparation de chlorophénols caractérisé en ce que l'on déshydrochlore thermiquement à une température supérieure à 120° C une chlorocyclohexanone de formule générale:

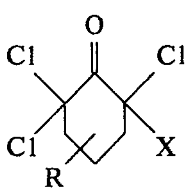

(I)

dans laquelle X représente un atome d'hydrogène ou un atome de chlore, et R représente au moins un radical choisi parmi le groupe comprenant H, les radicaux alkyle ayant de 1 à 5 atomes de carbone en présence d'un catalyseur de formule générale:

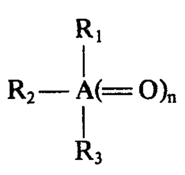

(II)

dans laquelle:

- A représente le phosphore ou l'arsenic;
- $R_1$ et $R_2$ identiques ou différents représentent un atome d'hydrogène ou un radical choisi parmi le groupe comprenant les radicaux alkyle contenant de 1 à 12 atomes de carbone, les radicaux phényle et les radicaux:

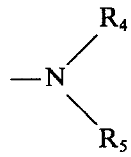

où $R_4$ et $R_5$ identiques ou différents représentent H ou un radical alkyle ayant de 1 à 5 atomes de carbone;
- $R_3$ représente un radical choisi parmi le groupe comprenant les radicaux alkyle contenant de 1 à 12 atomes de carbone, les radicaux phényle et les radicaux:

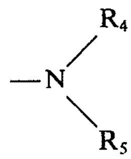

où $R_4$ et $R_5$ ont la même signification que ci-dessus;
- n est égal à 0 ou 1.

2. Procédé selon la revendication 1, caractérisé en ce que dans la formule II $R_1$ et $R_2$ identiques ou différents représentent un atome d'hydrogène ou un radical choisi parmi le groupe comprenant les radicaux alkyle ayant de 1 à 4 atomes de carbone et le radical phényle, $R_3$ représentant un radical choisi parmi le groupe comprenant les radicaux alkyle ayant de 1 à 4 atomes de carbone et le radical phényle.

3. Procédé selon la revendication 2 caractérisé en ce que le composé de formule II est la triphénylphosphine.

4. Procédé selon la revendication 2 caractérisé en ce que le composé de formule II est le triphénylarsine.

5. Procédé selon l'une quelconque des revendications précédentes caractérisé en ce que la quantité de composé de formule II utilisée est comprise entre 0,1 et 10% en poids du composé de formule I.

6. Procédé selon la revendication 5 caractérisé en ce que la quantité de composé de formule II est comprise entre 0,5% et 2%.

7. Procédé selon la revendication 1 caractérisé en ce que on décompose le composé de formule I à une température comprise entre 140 et 180° C.

8. Procédé selon la revendication 7, caractérisé en ce que la température est égale à environ 160° C.

9. Procédé selon la revendication 1, caractérisé en ce que le composé de formule I est la tétrachloro-2,2,6,6 cyclohexanone.

10. Procédé selon la revendication 1, caractérisé en ce que le composé de formule I est la trichloro-2,26 cyclohexanone.

**Patentansprüche**

1. Verfahren zur Herstellung von Chlorphenolen, dadurch gekennzeichnet, daß man bei einer höheren Temperatur als 120° C ein Chlorcyclohexanon der allgemeinen Formel

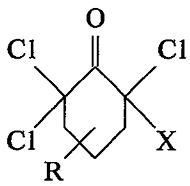

(I)

thermisch dehydrochloriert, worin X ein Wasserstoffatom oder ein Chloratom darstellt und R zumindest einen Rest darstellt, der unter der Gruppe ausgewählt ist aus H, Alkylgruppen mit 1 bis 15 Kohlenstoffatomen in Gegenwart eines Katalysators der allgemeinen Formel

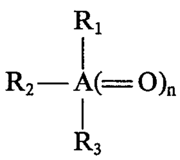

(II)

worin

- A Phosphor oder Arsen darstellt;
- $R_1$ und $R_2$, die gleich oder verschieden sein können, ein Wasserstoffatom oder einen Rest darstellen, der ausgewählt ist aus der Gruppe aus Alkylresten mit 1–12 Kohlenstoffatomen, Phenylresten und den Resten:

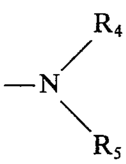

worin $R_4$ und $R_5$, die gleich oder verschieden sein können, für H oder einen Alkylrest mit 1–5 Kohlenstoffatomen stehen;
- $R_3$ einen Rest darstellt, der ausgewählt ist aus der Gruppe aus Alkylresten mit 1–12 Kohlenstoffatomen, Phenylresten und den Resten:

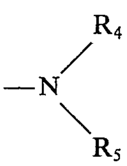

worin $R_4$ und $R_5$ die oben angegebenen Bedeutungen haben;
- n = 0 oder 1 ist.

2. Verfahren nach Anspruch 1, dadurch gekennzeichnet, daß in der Formel II $R_1$ und $R_2$, die gleich oder verschieden sein können, ein Wasserstoffatom oder einen Rest darstellen, der ausgewählt ist aus der Gruppe aus Alkylresten mit 1–4 Kohlenstoffatomen und dem Phenylrest, $R_3$ einen Rest darstellt, der ausgewählt ist aus der Gruppe aus Alkylresten mit 1–4 Kohlenstoffatomen und dem Phenylrest.

3. Verfahren nach Anspruch 2, dadurch gekennzeichnet, daß die Verbindung der Formel II Triphenylphosphin ist.

4. Verfahren nach Anspruch 2, dadurch gekennzeichnet, daß die Verbindung der Formel II Triphenylarsin ist.

5. Verfahren nach einem der vorhergehenden Ansprüche, dadurch gekennzeichnet, daß die eingesetzte Menge der Verbindung der Formel II zwischen 0,1 und 10 Gew.-% der Verbindung der Formel I beträgt.

6. Verfahren nach Anspruch 5, dadurch gekennzeichnet, daß die Menge der Verbindung der Formel II zwischen 0,5% und 2% ausmacht.

7. Verfahren nach Anspruch 1, dadurch gekennzeichnet, daß man die Verbindung der Formel I bei einer Temperatur zwischen 140 und 180° C zersetzt.

8. Verfahren nach Anspruch 7, dadurch gekennzeichnet, daß die Temperatur ungefähr 160° C ist.

9. Verfahren nach Anspruch 1, dadurch gekennzeichnet, daß die Verbindung der Formel I 2,2,6,6-Tetrachlorcyclohexanon ist.

10. Verfahren nach Anspruch 1, dadurch gekennzeichnet, daß die Verbindung der Formel I 2,2,6-Trichlorcyclohexanon ist.

**Claims**

1. Process for the preparation of chlorophenols, characterised in that a chlorocyclohexanone of the general formula:

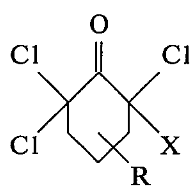

(I)

in which X represents a hydrogen atom or a chlorine atom and R represents at least one radical chosen from amongst the group comprising H and alkyl radicals having from 1 to 5 carbon atoms, is thermally dehydrochlorinated at a temperature above 120°C, in the presence of a catalyst of the general formula:

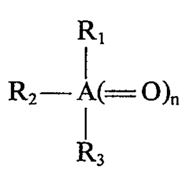

(II)

in which: A represents phosphorus or arsenic, $R_1$ and $R_2$, which are identical or different, represent

a hydrogen atom or a radical chosen from amongst the group comprising alkyl radicals containing from 1 to 12 carbon atoms, phenyl radicals and the radicals:

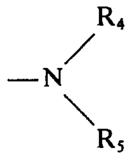

in which $R_4$ and $R_5$, which are identical or different, represent H or an alkyl radical having from 1 to 5 carbon atoms, $R_3$ represents a radical chosen from amongst the group comprising alkyl radicals containing from 1 to 12 carbon atoms, phenyl radicals and the radicals:

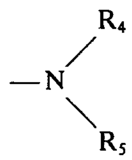

in which $R_4$ und $R_5$ have the same meaning as above, and n is equal to 0 or 1.

2. Process according to Claim 1, characterised in that, in the formula II, $R_1$ and $R_2$, which are identical or different, represent a hydrogen atom or a radical chosen from amongst the group comprising alkyl radicals having from 1 to 4 carbon atoms and the phenyl radical, $R_3$ representing a radical chosen from amongst the group comprising alkyl radicals having from 1 to 4 carbon atoms and the phenyl radical.

3. Process according to Claim 2, characterised in that the compound of the formula II is triphenylphosphine.

4. Process according to Claim 2, characterised in that the compound of the formula II is triphenylarsine.

5. Process according to any one of the preceding claims, chracterised in that the amount of compound of the formula II used is between 0.1 and 10% by weight of the compound of the formula I.

6. Process according to Claim 5, characterised in that the amount of compound of the formula II is between 0.5% and 2%.

7. Process according to Claim 1, characterised in that the compound of the formula I is decomposed at a temperature between 140 and 180°C.

8. Process according to Claim 7, characterised in that the temperature is equal to about 160°C.

9. Process according to Claim 1, characterised in that the compound of the formula I is 2,2,6,6-tetrachlorocyclohexanone.

10. Process according to Claim 1, characterised in that the compound of the formula I is 2,2,6-trichlorocyclohexanone.